Europäisches Patentamt

European Patent Office

Office européen des brevets

(11) Veröffentlichungsnummer: **0 076 779**
**A2**

(12) **EUROPÄISCHE PATENTANMELDUNG**

(21) Anmeldenummer: **82810389.5**

(22) Anmeldetag: **20.09.82**

(51) Int. Cl.³: **B 09 B 5/00**

(30) Priorität: **18.09.81 CH 6026/81**

(43) Veröffentlichungstag der Anmeldung:
**13.04.83 Patentblatt 83/15**

(84) Benannte Vertragsstaaten:
**AT BE DE FR GB IT NL SE**

(71) Anmelder: **FOLLI BETEILIGUNGSANSTALT**
**Cholmad 141**
**FL-9491 Nendeln(LI)**

(72) Erfinder: **Frey, Heinrich**
**Verenastrasse 19**
**CH-8038 Zürich(CH)**

(54) **Verfahren zur Verarbeitung von Müll und Müllbestandteilen.**

(57) Zur vollen Ausnützung für die Weiterverwertung von Müll, und zwecks Verhinderung einer vorzeitigen Verrottung desselben, wird dem Müll verrottungshemmendes Material zugeführt, welches aus Gasen, z.B. Ozon, Dämpfen von chemischen Mitteln, Imprägnierungs- oder Beschichtungsmitteln, bestehen kann.

Diese Zuführung erfolgt bereits beim Einsammeln des Mülls und wird praktisch bei jedem Arbeitsgang oder jeder Lagerung erneut durchgeführt. Der Müll wird zu einem faserigen Endprodukt verarbeitet, das als technisch verwendbarer Ersatzstoff dient.

EP 0 076 779 A2

Folli Beteiligungsanstalt, Nendeln
(Liechtenstein)

Verfahren zur Verarbeitung von Müll und Müllbestandteilen

Die Erfindung betrifft ein Verfahren zur Verarbeitung
von Müll und Müllbestandteilen gemäss Oberbegriff des Patentanspruch 1.

Es sind zahlreiche Verfahren bekannt, mit welchen Haushaltmüll zur Gewinnung von Fasern als Holz- oder Füllmaterial-
Ersatz verarbeitet wird.

Die Ausbeute an solchen Fasern ist sehr unterschiedlich
und zum Teil gering, was darauf hinweist, dass die bisher bekannten Verfahren verschiedene Mängel aufweisen.

Untersuchungen haben ergeben, dass eine grösstmögliche
Ausbeute an verwendbaren Fasern erzielbar ist, sofern der anfallende Müll schnellstens stabilisiert, d.h. wenn bereits
eine beginnende Verrottung möglichst unterbunden wird. Ebenso
soll während der weiteren Behandlung des Mülls keine neue
Verrottung aufkommen.

Aufgabe der Erfindung ist die Ausbeute von anfallendem
Müll zu steigern und gleichzeitig das Endprodukt für die Weiterverwertung zu verbessern.

Erfindungsgemäss wird die Aufgabe beim eingangs genannten
Verfahren durch die im kennzeichnenden Teil des Patentanspruch
1 enthaltenen Merkmale gelöst.

Demgemäss soll nun der Müll in möglichst zahlreichen Verarbeitungsstufen oder auch zwischen solchen Stufen, z.B. bei
der Zwischenlagerung, einer stabilisierenden Behandlung unterzogen werden. Dabei kann auch die Endlagerung oder Endverpackung der produzierten Fasern mit einbezogen werden, sofern diese nicht unmittelbar verbraucht werden.

Zur Stabilisierung können Gase, Dämpfe von chemischen Mitteln, Imprägnierung und Beschichtung, angewendet werden. ·

Es ist bekannt, den Müll oder Bestandteile davon in Trocknungseinrichtungen zu entfeuchten. Insofern dabei Hitze angewendet wird, erfolgt eine Hygienisierung, wodurch eine momentane Stabilisierung erreicht wird. Ueblicherweise erfolgt diese Behandlung jedoch erst einige Zeit nach der Mülleinsammlung, nach mehrmaliger Zwischenlagerung und Zerkleinerung. Diese zeitliche Verzögerung ergibt aber bereits Qualitätseinbussen und eine Reduktion der möglichen gesamten Faserausbeute infolge bereits angelaufener Verfaulung, was mit dem erfindungsgemässen Verfahren verhindert wird.

Der eingesammelte Müll soll also sofort verrottungshemmenden Einflüssen unterzogen werden und diese Behandlung ist zu erneuern, so oft in der Verarbeitung des Mülls oder dessen Bestandteilen ein Unterbruch, sei es aus betrieblichen oder anderen Gründen, stattfindet. Solange das endgültige Produkt, z.B. in Faserform, nicht vollständig herausgearbeitet ist, besteht die Gefahr einer neu beginnenden Verrottung.

Aus diesem Grunde werden vorteilhafterweise bereits während den einzelnen Verarbeitungsstufen verrottungshemmende Vorkehren getroffen, wie dies in der Folge beispielsweise beschrieben wird, bis das endgültig aus dem Müll herauszuarbeitende Produkt, d.h. die Faser, erzielt ist.

Da der Müll aus einer Vielzahl verschiedener Komponenten besteht, welche sich zur Faserherstellung eignen, jedoch die Behandlung, entsprechend den verschiedenen Materialien, unterschiedlich sein sollte, ist eine Trennung mit Mitteln bekannter Art vorgesehen.

Dies ermöglicht, z.B. reine Kunststoffteile zu beizen oder zu beschichten um ihre Benetzungsfähigkeit zu erhöhen, womit auch solche Teile in einem Endprodukt, z.B. einem Pressling, dessen Festigkeit dadurch erhöht wird, eingebaut werden können.

Im Gegensatz dazu stehen vegetabile Fasern, die enorm saugfähig sind und damit bei der Endverarbeitung einen übermässigen Anteil von Bindemitteln beanspruchen. Durch eine Imprägnierung kann die Saugfähigkeit auf das bei reinen Holzfasern übliche Mass reduziert werden, so dass hieraus, im Vergleich zu Holz, keine erhöhten Bindemittelkosten entstehen.

Eine weitere Schwierigkeit in der Fasererzeugung aus Müll liegt darin, dass sich schwer lösbare Knäuel bilden, welche die weitere Verarbeitung erschweren oder gar verunmöglichen. Die Imprägnierung oder Beschichtung verhindert die Knäuelbildung, denn die Fasern können sich nicht mehr ineinanderhaken.

Mit der Behandlung der Müllkomponenten kann auch gleichzeitig ein Färbeprozess verbunden werden, der dem Endprodukt ein einheitliches Aussehen verleiht oder zur Kennzeichnung dienen kann.

Entsprechend den Ansprüchen an das Endprodukt kann dieses als Ganzes oder Fraktionen davon, verschiedenen Behandlungsarten unterzogen werden, z.B. beizen, färben, imprägnieren, fixieren, bedampfen, und/oder beschichten.

Sofern die aus dem Müll herausgearbeiteten Fasern gelagert werden müssen, kann dies in Silos oder Behältern anderer Art, unter Schutzgaseinwirkung oder Dämpfen, geschehen. Bei Sackabpackung kann auch das Vakuum-Verfahren angewendet werden, wobei dem Sackinhalt jeder Sauerstoff entzogen wird.

Als fäulnishemmende Mittel können, ausser der bereits erwähnten Hitze, auch Ozongas sowie chemische Mittel bekannter Art oder deren Dämpfe, angewendet werden.

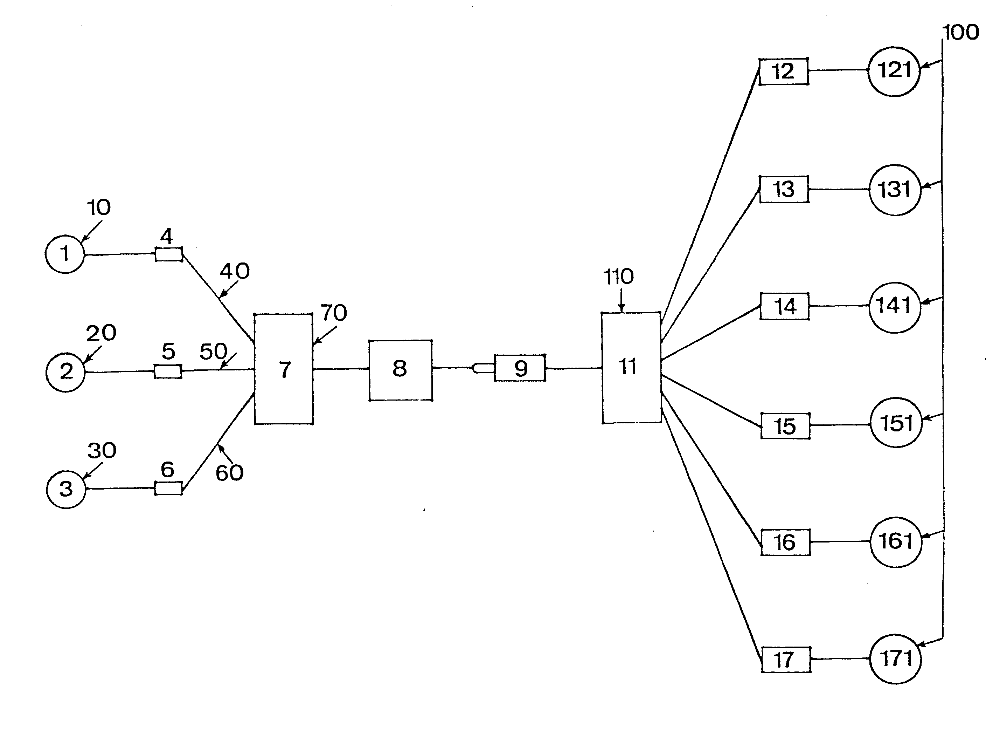

Auf der Zeichnung ist ein beispielsweises Arbeitsschema des erfindungsgemässen Verfahrens dargestellt.

Mit 1 - 3 sind Sammelstellen für den Müll bezeichnet. Dies können Presscontainer oder andere Mittel sein. Bei jedem neuen Mülleinschub wird, je über eine Gaspatrone 10, 20, 30, eine kleine Menge Gas eingeblasen. Der gefüllte Container wird durch einen leeren Container ausgetauscht und damit auch die Gaspatrone. 4 - 6 sind die Transportmittel. Sofern der Müll umgeladen werden muss, wird auch hier eine weitere Begasung 40, 50, 60 vorgenommen. Der Müll wird einem zentralen Eingangsbunker 7 einer Sammelstelle oder eines Werkes zugeführt. In der Sammelstelle wird der Müll vor der Weiterleitung event. zerkleinert. In jedem Fall wird auch hier der Verfall des Mülls durch Gaseintragung erneut gestoppt. Sofern es sich um den Eingangsbunker des Werkes handelt, wird vorzugsweise Ozongas eingeführt und durch Unterdruck übelriechende Luft abgesogen, gereinigt oder verbrannt. Bei 8 erfolgt die Zerkleinerung und die Vorsortierung, wie z.B. die Aussortierung von Steinen, Glas und Metallen. In 9 findet nun die Trocknung durch Hitze oder Vakuum statt und bei 11 die weitere Behandlung, z.B. durch Ozoneintrag 110 und/oder den anderen, unter den Ziffern 12 - 17 erwähnten Möglichkeiten. Anschliessend erfolgt die Aufteilung in verschiedene Fraktionen.

Bei 11 kann aber auch vor der endgültigen Behandlung eine Aufteilung des Zwischenproduktes erfolgen, worauf die einzelnen Gruppen spezifisch behandelt werden, wie 12 färben, 13 beizen, 14 imprägnieren, 15 fixieren, 16 bedampfen, 17 beschichten. Danach wird das Endprodukt in den Bunkern 121, 131, 141, 151, 161, 171 gelagert. In diesen Bunkern liegen die Fasern wiederum unter einem Schutzgas 100, so dass sie bis zur weiteren endgültigen Verwendung keinerlei, ihre Qualität verschlechternden Veränderung mehr unterworfen sind.

Die Faserbildung erfolgt im Laufe der Bearbeitung des Mülls, welcher z.B. durch Schlag-, Hack- und Zerreissmaschinen getrieben wird, wobei sich die Fasern bilden. Bei jedem Brechprozess wird neues Material, z.B. an der Bruchfläche, freigelegt, so dass stets neues fäulnishemmendes Material zugeführt werden muss.

- 5 -

Bei der Behandlung gemäss 12 - 17 oder der Bunkerung, kann auch eine Aromatisierung zwecks angenehmerer Endverarbeitung erfolgen. Dasselbe kann im Falle einer Vakuumsackverpackung vorgenommen werden, wodurch das Endprodukt durch längere Lagerung und Transporte über grosse Strecken, nicht beeinflusst wird.

Die Anwendung dieses Verfahrens ermöglicht eine hundertprozentige Ausnützung des Mülls für die Wiederverwertung. Abgesehen vom entfernten Wasser, erhält man, ausser dem Fasermaterial, Metalle, wie Messing, Aluminium und wenig Eisen; Schwerteile, wie Blei, Steine; Glassplitter enthaltenden Sand.

3981H

- 01 -

Folli Beteiligungsanstalt, Nendeln
(Liechtenstein)


P a t e n t a n s p r ü c h e

1.    Verfahren zur Verarbeitung von Müll und Müllbestandteilen, vorzugsweise Haushaltmüll, zu einem technisch verwendbaren Ersatzstoff, beispielsweise in Form von Fasern,
dadurch gekennzeichnet, dass das Müllmaterial beim Einsammeln und/oder während der Verarbeitung und/oder während der
Lagerung des Endproduktes unter Stabilisierungseinfluss gebracht wird.

2.    Verfahren nach Anspruch 1, dadurch gekennzeichnet, dass
die Stabilisierung durch Zuführung von verrottungshemmendem
Material, wie Gase, z.B. Ozon, Dämpfe von chemischen Mitteln,
Imprägnierungs- oder Beschichtungsmitteln, erfolgt.

3.    Verfahren nach Anspruch 1 oder 2, dadurch gekennzeichnet, dass die Stabilisierung durch Einwirkung verrottungshemmender Einflüsse, wie unter Druck- und unter Vakuumsetzung,
Wärme- oder Kältebehandlung oder Feuchtigkeitsentzug, erfolgt.

4.    Verfahren nach einem der Ansprüche 1 - 3, dadurch gekennzeichnet, dass die Lagerung des Endproduktes bis zu dessen Weiterverarbeitung unter Vakuum erfolgt.

5.    Verfahren nach einem der Ansprüche 1 - 4, wobei der
Müll zu Fasern verarbeitet wird, dadurch gekennzeichnet, dass
die Fasern gebeizt werden.

6.    Verfahren nach einem der Ansprüche 1 - 4, wobei der
Müll zu Fasern verarbeitet wird, dadurch gekennzeithnet, dass
die Fasern gefärbt werden.

**0076779**

7.      Verfahren nach einem der Ansprüche 1 - 4, wobei der
Müll zu Fasern verarbeitet wird, dadurch gekennzeichnet, dass
die Fasern imprägniert werden.

8.      Verfahren nach einem der Ansprüche 1 - 4, wobei der
Müll zu Fasern verarbeitet wird, dadurch gekennzeichnet, dass
die Fasern fixiert werden.

9.      Verfahren nach einem der Ansprüche 1 - 4, wobei der
Müll zu Fasern verarbeitet wird, dadurch gekennzeichnet, dass
die Fasern bedampft und/oder beschichtet werden.

3981H

0076779

1/1